# EUROPÄISCHE PATENTSCHRIFT

(11) **EP 3 013 936 B1**
(45) Veröffentlichungstag und Bekanntmachung des Hinweises auf die Patenterteilung: **15.03.2017**
(21) Anmeldenummer: 14731920.6
(22) Anmeldetag: 28.05.2014
(51) Int. Cl.: C12M 1/00, C12M 1/06, B01F 7/00, B01F 15/00, F16C 33/00

(54) **BEHÄLTER MIT EINEM WELLENGEHÄUSE**
RECEPTACLE COMPRISING A SHAFT HOUSING
RÉSERVOIR ÉQUIPÉ D'UN LOGEMENT D'ARBRE

(30) Priorität: 26.06.2013 DE 102013106680
(43) Veröffentlichungstag der Anmeldung: 04.05.2016
(73) Patentinhaber: Sartorius Stedim Biotech GmbH, 37079 Göttingen (DE)
(72) Erfinder: SEITZ, Reiner, 34298 Helsa (DE); SALZMANN, Rainer, 34212 Melsungen (DE); REGEN, Thomas, 37079 Göttingen (DE); HIELSCHER, Matthias, 37235 Hessisch Lichtenau (DE); HUSEMANN, Bernward, 37079 Göttingen (DE); ZEUCH, Stefan, 37079 Göttingen (DE); HUSEMANN, Ute, 37079 Göttingen (DE); GRELLER, Gerhard, 37085 Göttingen (DE)
(74) Vertreter: Schneider, Peter Christian
(86) Internationale Anmeldenummer: PCT/EP2014/061025
(87) Internationale Veröffentlichungsnummer: WO 2015/022099

(56) Entgegenhaltungen:
- DE-A1-102009 018 209
- DE-B4-102008 010 427
- US-A1- 2006 280 028

## Beschreibung

### Gebiet der Erfindung

Die Erfindung betrifft einen Behälter mit einem Wellengehäuse zur Durchführung einer Welle durch eine Wandung in den Behälterinnenraum, wobei die Welle mit einem Wellenführungsteil in dem Wellengehäuse über Kugellager gelagert und durch Dichtungen gegenüber dem Wellengehäuse abgedichtet ist.

### Stand der Technik

Aus der DE 10 2008 010 427 B4 ist ein Behälter mit einem Wellengehäuse zur Durchführung einer Welle durch eine Wandung in den Behälterinnenraum bekannt. Dabei wird die Welle mit einem Wellenführungsteil in dem Wellengehäuse über Kugellager gelagert und durch Dichtungen gegenüber dem Wellengehäuse abgedichtet.

Nachteilig bei dem bekannten Behälter, der sich insbesondere für Bioreaktoren mit flexibler Wandlung grundsätzlich bewährt hat, ist, dass Abrieb von Dichtlippen in den Behälterinnenraum eindringen kann. Bei Bewegungen des Behälters kann es zu einer ungleichmäßigen Belastung der Abdichtung kommen.

Weiterhin ist aus der US 2006/0280028 A1 ein Wellengehäuse zur Durchführung einer Welle durch eine Wandung in einen Behälterinnenraum bekannt. Dabei wird ein Wellenführungsteil in dem Wellengehäuse durch zwei Radialkugellager gelagert. Das Wellenführungsteil weist einen radial angeordneten ebenen Bund auf, der zu beiden Seiten durch Dichtungen abdichtbar ist.

Nachteilig dabei ist, dass zum Behälterinnenraum hin angeordnete Dichtungen durch eine zusätzliche Halterung am Wellengehäuse gehalten werden müssen. Weiterhin nachteilig bei dieser bekannten Wellendurchführung ist, dass die beiden Kugellager an dem den Behälterinnenraum abgewandten Ende des Wellenführungsteiles dicht beieinander angeordnet sind. Bei Bewegungen des Beutels bzw. des Behälters kann es dabei zu einer ungleichmäßigen Belastung der Abdichtung zwischen Wellengehäuse und Wellenführungsteil kommen.

Weiterhin ist aus der DE 10 2009 018 209 A1 ein als flexibler Beutel ausgebildeter Behälter mit einer Mischvorrichtung bekannt, die einen längenverstellbaren Mischerschaft aufweist. Der Mischerschaft ist dabei als eine Teleskopwelle ausgebildet, die in ihrer Längsrichtung teleskopartig verschiebbar ist.

### Aufgabenstellung

Es ist die Aufgabe der vorliegenden Erfindung, die bekannten Behälter mit einem Wellengehäuse zur Durchführung einer Welle durch eine Wandung so zu verbessern, dass einerseits das Eindringen von Abrieb der Dichtungen in den Behälterinnenraum und andererseits eine ungleichmäßige Belastung der Abdichtung kostengünstig vermieden wird.

### Darlegung der Erfindung

Diese Aufgabe wird in Verbindung mit den Merkmalen des Oberbegriffs von Anspruch 1 dadurch gelöst, dass das Wellengehäuse zum Behälterinnenraum hin ringkappenförmig ausgebildet ist und beabstandet zur Außenwandung eine Innenwandung aufweist, die über eine dem Behälterinnenraum zugewandte innere Stirnfläche mit der Außenwandung des Wellengehäuses verbunden ist, dass das Wellenführungsteil an seinem dem Behälterinnenraum zugewandten Ende eine Außenfläche aufweist, die gegenüber der Innenwandung des Wellengehäuses einen Spalt bildet, dass das Wellenführungsteil beabstandet und parallel zu seiner Außenfläche eine ringförmige Wandung aufweist, die das freie Ende der Innenwandung des Wellengehäuses kappenförmig übergreift, und dass die ringförmige Wandung gegenüber der Innenwandung des Wellengehäuses einerseits und gegenüber der Außenwandung des Wellengehäuses andererseits durch jeweils mindestens eine Dichtung abgedichtet ist, und dass das Wellenführungsteil in dem Wellengehäuse über ein erstes als Radialkugellager ausgebildetes Kugellager und über ein zweites als Axialkugellager ausgebildetes Kugellager gelagert ist.

Durch die ringkappenförmige Ausbildung des Wellengehäuses wird zuverlässig erreicht, dass kein Abrieb der Dichtungen in den Behälterinnenraum eindringen kann. Zugleich wird dies ohne die Verwendung und Montage eines zusätzlichen Teiles kostengünstig erreicht. Durch die ringkappenförmige Ausbildung des Gehäuseteiles kann in dem Gehäuseteil ein Axialkugellager angeordnet werden, auf dem sich das Wellenführungsteil mit seiner ringförmigen parallel zu seiner Außenfläche angeordneten Wandung in axialer Richtung abstützten kann. Das zweite beabstandete Radialkugellager führt in Kombination mit dem Axialkugellager zu einer gleichmäßigeren Belastung der Abdichtung auch bei Bewegungen eines als flexiblen Beutels ausgebildeten Behälters. Die Abdichtung der ringförmigen Wandung gegenüber der Innenwandung des Wellengehäuses einerseits und gegenüber der Außenwandung des Wellengehäuses andererseits durch jeweils mindestens eine Dichtung führt zu einer Art Labyrinthdichtung, die sich als besonders günstig erwiesen hat.

Gemäß einer bevorzugten Ausführungsform der Erfindung ist die ringförmige Wandung des Wellenführungsteils gegenüber der Innenwandung des Wellengehäuses in Längsrichtung der Welle durch zwei hintereinander angeordnete Dichtungen abgedichtet. Die unmittelbar hintereinander angeordneten Dichtungen vergrößern durch ihre zusätzliche Labyrinthwirkung die Abdichtung.

Nach einer weiteren bevorzugten Ausführungsform der Erfindung sind die Dichtungen als Radialwellendichtringe mit mindestens einer Dichtlippe ausgebildet. Durch die Anordnung von Dichtlippen wird bei guter Abdichtung die Reibung vermindert und dadurch auch ein evtl. Abrieb reduziert.

Nach einer weiteren bevorzugten Ausführungsform der Erfindung ist das Wellenführungsteil fest mit einer in den Behälterinnenraum hineinragenden Welle verbunden. Die feste Verbindung zwischen Wellenführungsteil und der Welle vermeidet zum einen Abdichtprobleme zwischen Wellenführungsteil und Welle; hat aber den Nachteil, dass die Welle gegenüber dem Führungsteil nicht längsverschiebbar ist.

Gemäß einer weitern bevorzugten Ausführungsform der Erfindung weist das Wellenführungsteil eine Aufnahmeöffnung zur Aufnahme einer in den Behälterinnenraum hineinragenden Welle auf. Die in den Behälterinnenraum hineinragende Welle kann dabei als eine Welle ausgebildet sein, die mindestens abschnittsweise von einem Faltenbalg dichtend umhüllt ist und an ihrem dem Wellengehäuse zugewandten Ende mit dem Wellenführungsteil verbunden ist.

Durch die Verwendung eines Faltenbalgs wird das Abdichtproblem zwischen Führungsteil und Welle zuverlässig gelöst.

Nach einer weiteren bevorzugten Ausführungsform der Erfindung ist die Welle mehrteilig aus Wellensegmenten in einer festgelegten Reihenfolge ausgebildet, wobei die Wellensegmente im getrennten Zustand unverlierbar durch ein Halteelement verbunden sind. Das Halteelement kann beispielsweise ein Seil sein. Dadurch besitzt die Welle trennbare aber unverlierbare Einzelteile.

Gemäß einer weiteren bevorzugten Ausführungsform der Erfindung ist die Welle als eine Teleskopwelle ausgebildet.

Durch die in dem Behälterinnenraum hineinragende Teleskopwelle wird einerseits die Flexibilität erhöht und zum anderen wird, wie oben dargestellt, durch die Verwendung des Faltenbalgs das Abdichtproblem zwischen Führungsteil und Welle zuverlässig gelöst.

Nach einer weiteren bevorzugten Ausführungsform der Erfindung wird die Welle an ihrem dem Wellengehäuse abgewandten Ende in einer am Behälterboden angeordneten Führung geführt. Die Führung am Behälterboden trägt ebenfalls zu einer gleichmäßigeren Belastung der Welle und damit der Abdichtung zwischen Welle und Wellengehäuse bei.

Gemäß einer weiteren bevorzugten Ausführungsform der Erfindung besteht die Führung aus einem am Behälterboden angeordneten festen Führungsteil und einem drehbaren Aufnahmeteil für das Ende der Welle, wobei zwischen Aufnahmeteil und Führung mindestens ein Kugellager angeordnet ist. Die Anordnung des Kugellagers vermindert die Reibung und die Anordnung des Aufnahmeteiles erleichtert die Montage.

Nach einer weiteren Ausführungsform der Erfindung ist die Welle als eine Rührerwelle mit mindestens einem Rührer ausgebildet, an deren freies äußeres Ende eine Antriebswelle eines Antriebs ankoppelbar ist.

Bevorzugt ist der Behälter als ein Einwegbioreaktor mit flexiblen Wänden ausgebildet.

Weitere Merkmale und Vorteile der Erfindung ergeben sich aus der nachfolgenden speziellen Beschreibung und den Zeichnungen.

### Kurzbeschreibung der Zeichnungen

Es zeigen:

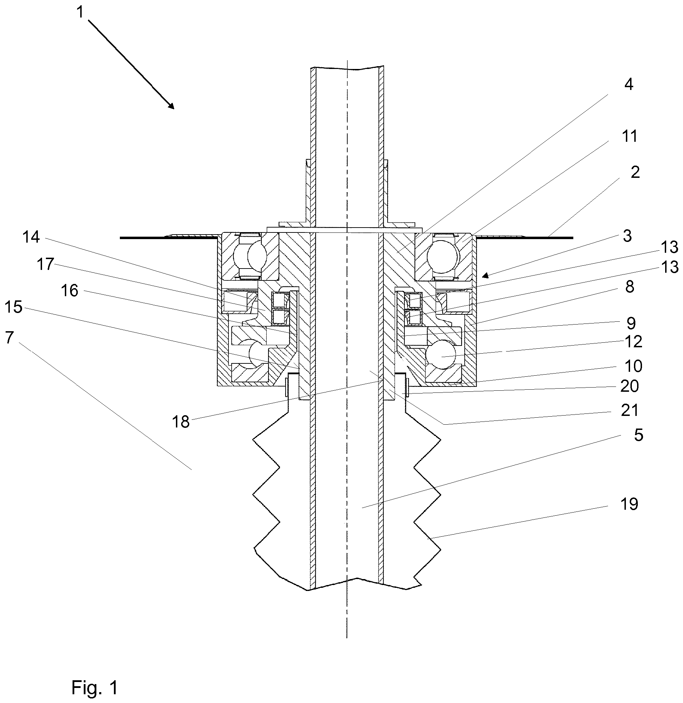

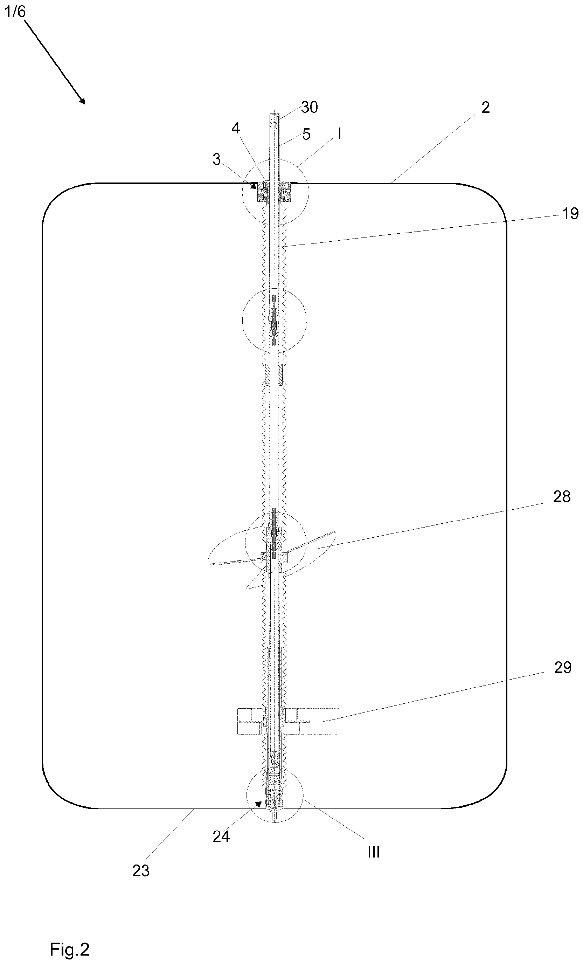

- Figur 1:: eine Seitenansicht im Schnitt und Ausriss eines Behälters mit einem Wellengehäuse, Wellenführungsteil, Welle und Faltenbalg als vergrößerte Darstellung der Einzelheit I von Figur 2;
- Figur 2:: eine Seitenansicht im Schnitt eines Behälters in einer Ausbildung als Bioreaktor mit Wellengehäuse, Wellenführungsteil, Welle als Rührerwelle mit Faltenbälgen und zwei Rührern und

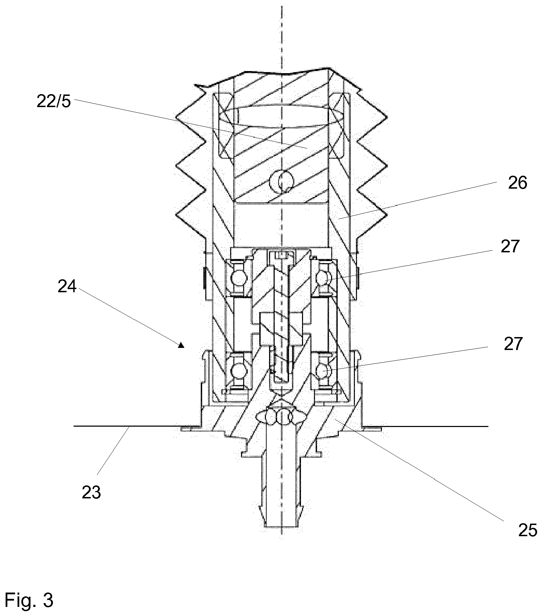

- Figur 3:: eine vergrößerte Darstellung der Einzelheit III von Figur 2.

### Beschreibung bevorzugter Ausführungsformen

Ein Behälter 1 besteht im Wesentlichen aus einer Wandung 2, einem Wellengehäuse 3, einem Wellenführungsteil 4 und einer Welle 5.

Der Behälter 1 weist die Wandung 2 auf, die im Ausführungsbeispiel als flexible Wandung eines Bioreaktors 6 ausgebildet ist. Die flexible Wandung 2 ist in vertikaler Richtung oben zur Durchführung der Welle 5 fest mit dem Wellengehäuse 3 verbunden.

Das Wellengehäuse 3 ist zu dem Behälterinnenraum 7 hin ringkappenförmig ausgebildet und weist beabstandet zu ihrer vertikalen Außenwandung 8 eine ebenfalls vertikale Innenwandung auf, die über eine dem Behälterinnenraum 7 zugewandte etwa horizontale Stirnfläche 10 mit der Außenwandung 8 des Wellengehäuses 3 verbunden ist. Die Welle 5 ist über das Wellenführungsteil 4 in dem Wellengehäuse 3 über Kugellager 11, 12 gelagert und durch Dichtungen 13, 14 gegenüber dem Wellengehäuse 3 abgedichtet. Das Wellenführungsteil 4 weist an seinem dem Behälterinnenraum 7 zugewandten Ende eine vertikale Außenfläche 15 auf, die gegenüber der Innenwandung 9 des Wellengehäuses 3 einen Spalt 16 bildet. Beabstandet und parallel zu seiner Außenfläche 15 weist das Wellenführungsteil 4 eine ringförmige Wandung 17 auf, die das freie Ende der Innenwandung 9 des Wellengehäuses 3 kappenförmig übergreift. Die ringförmige Wandung 17 ist gegenüber der Innenwandung 9 des Wellengehäuses 3 einerseits und gegenüber der Außenwandung 8 des Wellengehäuses 3 andererseits durch jeweils mindestens eine Dichtung 13, 14 abgedichtet. Im Ausführungsbeispiel ist die ringförmige Wandung 17 gegenüber der Innenwandung 9 durch zwei in vertikaler Richtung hintereinander angeordnete erste Dichtungen 13 abgedichtet. Die ringförmige Wandung 17 ist zur Außenwandung 8 des Wellengehäuses 3 hin durch eine zweite Dichtung 14 abgedichtet. Das Wellenführungsteil 4 ist in dem Wellengehäuse 3 in vertikaler Richtung oben durch das erste Kugellager 11, das als ein Radialkugellager ausgebildet ist und in vertikaler Richtung unten über das zweite Kugellager 12, das als ein Axialkugellager ausgebildet ist, gelagert. Die Dichtungen 13, 14 sind als Radialwellendichtringe mit jeweils einer Dichtlippe ausgebildet. Im Ausführungsbeispiel weist das Wellenführungsteil 4 eine Aufnahmeöffnung 18 oder einen Aufnahmekanal zur Aufnahme der in den Behälterinnenraum 7 hineinragenden Welle 5 auf. Die Welle 5 ist zum Wellengehäuse 3 hin von einem ersten Faltenbalg 19 dichtend umhüllt. Der Faltenbalg 19 ist an seinem oberen Ende 20 mit dem freien Ende 21 des Wellenführungsteil 4 verbunden. Entsprechend dem Ausführungsbeispiel wird die Welle 5 an ihrem dem Wellengehäuse 3 abgewandten unteren Ende 20 in einer am Behälterboden 23 angeordneten Führung 24 geführt. Die Führung 24 besteht aus einem am Behälterboden 23 angeordneten festen Führungsteil 25 und einem drehbaren Aufnahmeteil 26, in das das untere Ende 22 der Welle 5 einsetzbar ist. Das Aufnahmeteil 26 ist über zwei Kugellager 27, die im Ausführungsbeispiel beide als Radialkugellager ausgebildet sind, gelagert. Im Ausführungsbeispiel ist die Welle 5 als eine Rührerwelle mit einem ersten Rührer 28, der als ein Segment-Blattrührer ausgebildet ist und einem zweiten Rührer 29, der als ein Sechs-Blattrührer ausgebildet ist, ausgebildet. An das äußere freie Ende 30 der Welle 5 ist ein nicht dargestellter Antrieb ankoppelbar.
Natürlich stellen die in der speziellen Beschreibung diskutierten und in den Figuren gezeigten Ausführungsformen nur illustrative Ausführungsbeispiele der vorliegenden Erfindung dar. Dem Fachmann ist im Lichte der hiesigen Offenbarung ein breites Spektrum von Variationsmöglichkeiten an die Hand gegeben.

### Bezugszeichenliste

- 1: Behälter
- 2: Wandung von 1
- 3: Wellengehäuse
- 4: Wellenführungssteil
- 5: Welle
- 6: Bioreaktor
- 7: Behälterinnenraum
- 8: vertikale Außenwandung von 3
- 9: vertikale Innenwandung von 3
- 10: horizontale Stirnfläche von 3
- 11: erstes Kugellager, radial
- 12: zweites Kugellager, axial
- 13: Dichtung (2x)
- 14: Dichtung (2x)
- 15: vertikale Außenfläche von 4
- 16: Spalt
- 17: ringförmige Wandung von 4
- 18: Aufnahmeöffnung von 4
- 19: erster Faltenbalg
- 20: oberes Ende von 19
- 21: freies Ende von 4
- 22: unteres Ende von 5
- 23: Behälterboden von 1
- 24: Führung
- 25: Führungsteil von 24
- 26: Aufnahmeteil von 24
- 27: Kugellager von 24
- 28: erster Rührer
- 29: zweiter Rührer
- 30: äußeres freies Ende von 5

## Patentansprüche

1. Behälter (1) mit einem Wellengehäuse (3) zur Durchführung einer Welle (5) durch eine Wandung (2) in den Behälterinnenraum (7), wobei die Welle (5) mit einem Wellenführungsteil (4) in dem Wellengehäuse (3) über Kugellager (11, 12) gelagert und durch Dichtungen (13, 14) gegenüber dem Wellengehäuse (3) abgedichtet ist,
**dadurch gekennzeichnet,**
**dass** das Wellengehäuse (3) zum Behälterinnenraum (7) hin ringkappenförmig ausgebildet ist und beabstandet zur Außenwandung (8) eine Innenwandung (9) aufweist, die über eine dem Behälterinnenraum (7) zugewandte innere Stirnfläche (10) mit der Außenwandung (8) des Wellengehäuses (3) verbunden ist, dass das Wellenführungsteil (4) an seinem dem Behälterinnenraum (7) zugewandten Ende (21) eine Außenfläche (15) aufweist, die gegenüber der Innenwandung (9) des Wellengehäuses einen Spalt (16) bildet,
**dass** das Wellenführungsteil (4) beabstandet und parallel zu seiner Außenfläche (15) eine ringförmige Wandung (17) aufweist, die das freie Ende der Innenwandung (9) des Wellengehäuses (3) kappenförmig übergreift, und dass die ringförmige Wandung (17) gegenüber der Innenwandung (9) des Wellengehäuses (3) einerseits und gegenüber der Außenwandung (8) des Wellengehäuses (3) andererseits durch jeweils mindestens eine Dichtung (13, 14) abgedichtet ist, und
**dass** das Wellenführungsteil (4) in dem Wellengehäuse (3) über ein erstes als Radialkugellager ausgebildetes Kugellager (11) und über ein zweites als Axialkugellager ausgebildetes Kugellager (12) gelagert ist.

2. Behälter nach Anspruch 1,
**dadurch gekennzeichnet,**
**dass** die ringförmige Wandung (17) des Wellenführungsteils (4) gegenüber der Innenwandung (9) des Wellengehäuses (3) in Längsrichtung der Welle (5) durch zwei hintereinander angeordnete Dichtungen (13) abgedichtet ist.

3. Behälter nach Anspruch 1 oder 2,
**dadurch gekennzeichnet,**
**dass** die Dichtungen (13, 14) als Radialwellendichtringe mit mindestens einer Dichtlippe ausgebildet sind.

4. Behälter nach einem der Ansprüche 1 bis 3,
**dadurch gekennzeichnet,**
**dass** das Wellenführungsteil (4) fest mit einer in den Behälterinnenraum (7) hineinragenden Welle (5) verbunden ist.

5. Behälter nach einem der Ansprüche 1 bis 3,
**dadurch gekennzeichnet,**
**dass** das Wellenführungsteil (4) eine Aufnahmeöffnung (18) zur Aufnahme einer in den Behälterinnenraum (7) hineinragenden Welle (5) aufweist.

6. Behälter nach Anspruch 5,
**dadurch gekennzeichnet,**
**dass** die in den Behälterinnenraum (7) hineinragende Welle (5) mindestens Abschnittsweise von einem Faltenbalg (19) dichtend umhüllt ist und an ihrem dem Wellengehäuse (3) zugewandten Ende (20) mit dem Wellenführungsteil (4) verbunden ist.

7. Behälter nach einem der Ansprüche 1 bis 6,
**dadurch gekennzeichnet,**
**dass** die Welle (5) mehrteilig aus Wellensegmenten in festgelegter Reihenfolge ausgebildet ist und
**dass** die Wellensegmente im getrennten Zustand unverlierbar durch ein Halteelement verbunden sind.

8. Behälter nach einem der Ansprüche 1 bis 6,
**dadurch gekennzeichnet,**
**dass** die Welle (5) als Teleskopwelle ausgebildet ist.

9. Behälter nach einem der Ansprüche 1 bis 8,
**dadurch gekennzeichnet,**
**dass** die Welle (5) an ihrem dem Wellengehäuse (3) abgewandten Ende (22) in einer am Behälterboden (23) angeordneten Führung (24) geführt wird.

10. Behälter nach Anspruch 9,
**dadurch gekennzeichnet,**
**dass** die Führung (24) aus einem am Behälterboden (23) angeordneten festen Führungsteil (25) und einem drehbaren Aufnahmeteil (26) für das Ende (22) der Welle (5) besteht und dass zwischen Aufnahmeteil (26) und Führung (24) mindestens ein Kugellager (27) angeordnet ist.

11. Behälter nach einem der Ansprüche 1 bis 8,
**dadurch gekennzeichnet,**
**dass** die Welle (5) als Rührerwelle mit mindestens einem Rührer (28, 29) ausgebildet ist, an deren freies äußeres Ende (30) eine Antriebswelle eines Antriebs ankoppelbar ist.

12. Behälter nach einem der Ansprüche 1 bis 11,
**dadurch gekennzeichnet,**
**dass** der Behälter (1) als Einwegbioreaktor mit flexiblen Wänden ausgebildet ist.

## Claims

1. A receptacle (1) comprising a shaft housing (3) for guiding a shaft (5) through a wall (2) into a receptacle interior (7), the shaft (5) together with the shaft guide part (4) being mounted in the shaft housing (3) by way of ball bearings (11, 12) and being sealed relative to the shaft housing (3) by seals (13, 14),
**characterized in that**
the shaft housing (3) has the form of an annular cap towards the receptacle interior (7) and has an inner wall (9) spaced apart from the outer wall (8), said inner wall (9) being connected to the outer wall (8) of the shaft housing (3) via an inner end face (10) facing towards the receptacle interior (7);
the shaft guide part (4), at its end (21) facing towards the receptacle interior (7), has an outer surface (15) separated by a gap (16) from the inner wall (9) of the shaft housing;
the shaft guide part (4) has an annular wall (17) spaced apart from and parallel to its outer surface (15), the annular wall extends cap-like over the free end of the inner wall (9) of the shaft housing (3), and the annular wall (17) is sealed in relation to the inner wall (9) of the shaft housing (3) on the one end and in relation to the outer wall (8) of the shaft housing (3) on the other end by at least one seal (13, 14) each; and the shaft guide part (4) is mounted in the shaft housing (3) by way of a first ball bearing (11) in the form of a radial ball bearing and byway of a second ball bearing (12) in the form of an axial ball bearing.

2. The receptacle according to Claim 1,
**characterized in that**
the annular wall (17) of the shaft guide part (4) is sealed toward the inner wall (9) of
the shaft housing (3) in the longitudinal direction of the shaft (5) by two seals arranged one behind the other.

3. The receptacle according to either of Claims 1 or 2,
**characterized in that**
the seals (13, 14) are radial shaft sealing rings, each of which has at least one sealing lip.

4. The receptacle according to any of Claims 1 to 3,
**characterized in that**
the shaft guide part (4) is permanently connected to the shaft (5) extending into the receptacle interior (7).

5. The receptacle according to any of Claims 1 to 3,
**characterized in that**
the shaft guide part (4) has a receptor opening (18) to receive the shaft (5) extending into the receptacle interior (7).

6. The receptacle according to Claim 5,
**characterized in that**
the shaft (5) extending into the receptacle interior (7) is encased at least in sections by a bellows (19) that creates a seal, and is connected to the shaft guide part (4) at its end (20) facing towards the shaft housing (3).

7. The receptacle according to Claim 1,
**characterized in that**
the shaft (5) comprises multiple shaft segments arranged in a specified sequence, and
the shaft segments, thanks to a retaining element, cannot be separated from one another when they are detached.

8. The receptacle according to any of Claims 1 to 6,
**characterized in that**
the shaft (5) is a telescoping shaft.

9. The receptacle according to any of Claims 1 to 8,
**characterized in that**
the shaft (5) is guided at its lower end (22) facing away from the shaft housing (3) in a guide (24) arranged on the receptacle floor (23).

10. The receptacle according to Claim 9,
**characterized in that**
the guide (24) comprises a fixed guide part (25) arranged on the receptacle floor (23), and a rotatable receiving part (26) for the end (22) of the shaft (5), and at least one ball bearing (27) is arranged between the receiving part (26) and the guide (24).

11. The receptacle according to any of Claims 1 to 8,
**characterized in that**
the shaft (5) is an agitator shaft with at least one agitator (28, 29) to the free outer end (30) of which can be coupled a drive shaft of a drive.

12. The receptacle according to any of Claims 1 to 11,
**characterized in that**
the receptacle (1) is a single-use bioreactor with flexible walls.

## Revendications

1. Réservoir (1) équipé d'un logement d'arbre (3) destiné au passage d'un arbre (5) à travers une paroi (2) jusque dans l'espace intérieur (7) du réservoir, sachant que l'arbre (5) avec un élément de guidage d'arbre (4) est monté dans le logement d'arbre (3) par l'intermédiaire de roulements à billes (11, 12) et qu'il il est étanchéifié par rapport au logement d'arbre (3) par des joints d'étanchéité (13, 14),
**caractérisé en ce que** le logement d'arbre (3) est réalisé en forme de calotte annulaire en direction de l'espace intérieur (7) du réservoir et présente, à distance de la paroi extérieure (8), une paroi intérieure (9) qui est reliée à la paroi extérieure (8) du logement d'arbre (3) par l'intermédiaire d'une face frontale intérieure (10) tournée vers l'espace intérieur (7) du réservoir,
**en ce que** l'élément de guidage d'arbre (4) présente, à son extrémité (21) tournée vers l'espace intérieur (7) du réservoir, une surface extérieure (15) qui forme un interstice (16) par rapport à la paroi intérieure (9) du logement d'arbre,
**en ce que** l'élément de guidage d'arbre (4) présente, à distance de sa surface extérieure (15) et parallèlement à celle-ci, une paroi annulaire (17) qui engage en recouvrement en forme de calotte l'extrémité libre de la paroi intérieure (9) du logement d'arbre (3), et **en ce que** la paroi annulaire (17) est étanchéifiée, par rapport à la paroi intérieure (9) du logement d'arbre (3) d'une part et par rapport à la paroi extérieure (8) du logement d'arbre (3) d'autre part, par au moins un joint d'étanchéité respectif (13, 14),
et **en ce que** l'élément de guidage d'arbre (4) est monté dans le logement d'arbre (3) par l'intermédiaire d'un premier roulement à billes (11) réalisé sous forme de roulement radial à billes et d'un deuxième roulement à billes (12) réalisé sous forme de roulement axial à billes.

2. Réservoir selon la revendication 1, **caractérisé en ce que** la paroi annulaire (17) de l'élément de guidage d'arbre (4) est étanchéifiée par rapport à la paroi intérieure (9) du logement d'arbre (3), dans la direction longitudinale de l'arbre (5), par deux joints d'étanchéité (13) disposés l'un derrière l'autre.

3. Réservoir selon la revendication 1 ou 2, **caractérisé en ce que** les joints d'étanchéité (13, 14) sont réalisés sous forme de bagues radiales d'étanchéité d'arbre dotées d'au moins une lèvre d'étanchéité.

4. Réservoir selon l'une des revendications 1 à 3, **caractérisé en ce que** l'élément guidage d'arbre (4) de est fixement relié à un arbre (5) s'enfonçant dans l'espace intérieur (7) du réservoir.

5. Réservoir selon l'une des revendications 1 à 3, **caractérisé en ce que** l'élément de guidage d'arbre (4) présente une ouverture réceptrice (18) destinée à recevoir un arbre (5) s'enfonçant dans l'espace intérieur (7) du réservoir.

6. Réservoir selon la revendication 5, **caractérisé en ce que** l'arbre (5) s'enfonçant dans l'espace intérieur (7) du réservoir est enveloppé en étanchéité au moins sectoriellement par un soufflet (19) et est, à son extrémité (20) tournée vers le logement d'arbre (3), relié à l'élément de guidage d'arbre (4).

7. Réservoir selon l'une des revendications 1 à 6, **caractérisé en ce que** l'arbre (5) est réalisé en plusieurs parties consistant en des segments d'arbre dans un ordre de succession défini,
et **en ce que** les segments d'arbre, lorsqu'ils sont séparés, sont reliés de manière imperdable par un élément de retenue.

8. Réservoir selon l'une des revendications 1 à 6, **caractérisé en ce que** l'arbre (5) est réalisé sous forme d'arbre télescopique.

9. Réservoir selon l'une des revendications 1 à 8, **caractérisé en ce que** l'arbre (5) est, à son extrémité (22) opposée au logement d'arbre (3), guidé dans un guide (24) disposé sur le fond (23) du réservoir.

10. Réservoir selon la revendication 9, **caractérisé en ce que** le guide (24) est constitué d'une partie de guide fixe (25) disposée sur le fond (23) du réservoir et d'une partie réceptrice rotative (26) pour l'extrémité (22) de l'arbre (5), et **en ce qu'**au moins un roulement à billes (27) est disposé entre la partie réceptrice (26) et le guide (24).

11. Réservoir selon l'une des revendications 1 à 8, **caractérisé en ce que** l'arbre (5) est réalisé sous forme d'arbre agitateur équipé d'au moins un agitateur (28, 29), à l'extrémité extérieure libre (30) duquel peut être accouplé un arbre menant d'un entraînement.

12. Réservoir selon l'une des revendications 1 à 11, **caractérisé en ce que** le réservoir (1) est réalisé sous forme de bioréacteur à usage unique à parois flexibles.
